# EUROPEAN PATENT APPLICATION

(11) **EP 2 522 380 A1**
(43) Date of publication of application: **14.11.2012**
(21) Application number: 11165885.2
(22) Date of filing: 12.05.2011
(51) Int. Cl.: A61M 5/00, B65D 83/02, B65D 85/24

(54) **Needle assembly storage array**

(71) Applicant: Sanofi-Aventis Deutschland GmbH, 65929 Frankfurt am Main (DE)
(72) Inventor: The designation of the inventor has not yet been filed

(57) **Abstract**

Described is a needle assembly storage array (1) comprising a first needle assembly storage compartment; a second needle assembly storage compartment; and at least one connector (3) formed between the first needle assembly storage compartment and the second needle assembly storage compartment. The at least one connector (3) includes a break point (4) for separating the first needle assembly storage compartment and the second needle assembly storage compartment.

## Description

### Technical Field

The invention relates to a needle assembly storage array for storing needle assemblies.

### Background of the Invention

Patients suffering from diseases like diabetes have to frequently self-administer injections. Injection devices like auto-injectors or pen injectors have been developed to facilitate self-administering injections. Typically, such injection devices are disposable or reusable and are refitted with sterile injection needle assemblies to minimize the risk of infections.

Portable needle storage devices like needle magazines or needle dispensers contain a plurality of such sterile injection needle assemblies that are adapted to be mounted to the injection devices. The needle storage devices supplement the injection devices to facilitate safe self-administration of the medicament.

### Summary of the Invention

It is an object of the present invention to provide an improved means for storing needle assemblies.

In an exemplary embodiment, a needle assembly storage array comprises a first needle assembly storage compartment, a second needle assembly storage compartment, and at least one connector formed between the first needle assembly storage compartment and the second needle assembly storage compartment. The at least one connector includes a break point for separating the first needle assembly storage compartment and the second needle assembly storage compartment.

In an exemplary embodiment, openings of the first and second needle assembly storage compartments are disposed in a first plane and the break point is disposed in a second plane. The first plane may be substantially perpendicular to the second plane. In another exemplary embodiment, the first needle assembly storage compartment is inverted relative to the second needle assembly storage compartment.

In an exemplary embodiment, the connector comprises a first portion coupled to the first needle assembly storage compartment and a second portion coupled to the second needle assembly storage compartment. The break point may be formed between the first portion and the second portion.

In an exemplary embodiment, the connector includes at least one narrowed portion adjacent the break point.

In an exemplary embodiment, the first and second needle assembly storage compartments are made of a first material and the connector is made of a second material. The second material may be one of polystyrene, polycarbonate, and polymethyl methacrylate.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
Figure 1 shows a perspective view of a needle assembly storage array according to an exemplary embodiment of the present invention,
Figure 2 shows a perspective view of a section of a needle assembly storage array according to an exemplary embodiment of the present invention, and
Figure 3 shows a perspective view of a needle assembly storage array according to an exemplary embodiment of the present invention.

Corresponding parts are marked with the same reference symbols in all figures.

### Detailed Description

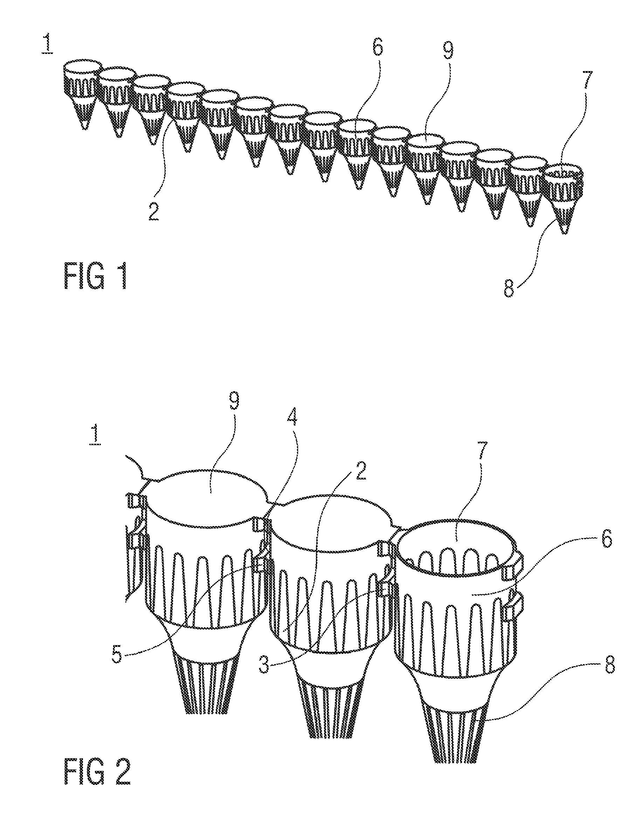

Figure 1 shows a perspective view of an exemplary embodiment of a needle assembly storage array 1 according to the present invention. The array 2 is formed of a plurality of interconnected needle assembly storage compartments 2, which are adapted to contain needle assemblies, such as those used with injection devices like pen injectors, autoinjectors, etc. A needle assembly generally comprises a needle hub adapted to fit on an injection device and a needle for delivering an injection. Each of the needle assembly storage compartments 2 comprises a proximal portion 6 and a distal portion 8. The proximal portion 6 may be sized and shaped to contain the needle hub and the distal portion 8 may be sized and shaped to contain the needle (or a portion thereof).

An opening 7 formed at the proximal portion 6 of the needle assembly storage compartment 2 may be covered by a removable seal 9. One seal 9 may be disposed on the opening 7 of each of the needle assembly storage compartments 2, or the seal 9 may be formed as a tape which extends across the openings 7 of multiple needle assembly storage compartments 2.

In the exemplary embodiment shown in Figure 1, the needle assembly storage compartments 2 are arranged in a row and all of the needle assembly storage compartments are oriented in the same manner. For example, the openings 7 of all of the needle assembly storage compartments 2 are disposed in a first plane, perpendicular to a longitudinal axis of the needle assembly storage compartments 2. Figure 2 shows an exemplary embodiment of an array 1 of needle assembly storage compartments 2. In this exemplary embodiment, consecutive needle assembly storage compartments are coupled by at least one connector 3. Those of skill in the art will understand that a connector may be formed between consecutive needle assembly storage compartments or between a second and third needle assembly storage compartments, a third and fourth, etc., such that separation at the connector may result in having a predetermined number of connected needle assembly storage compartments. In an exemplary embodiment, a patient or physician may separate needle assembly storage compartments in whichever increment he/she desires.

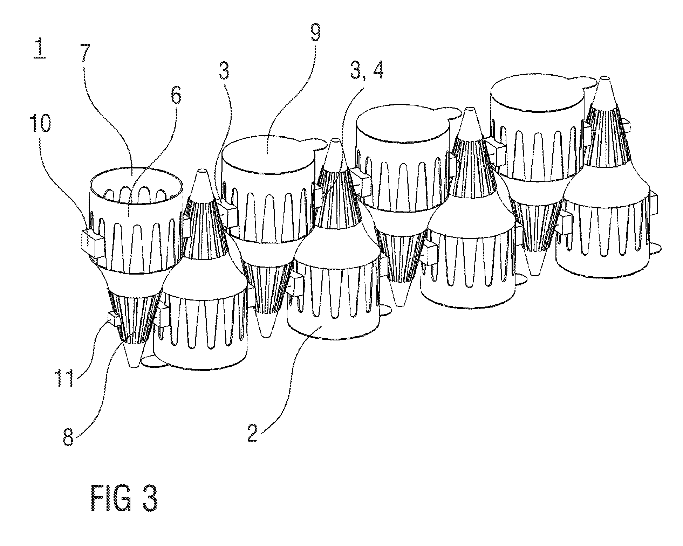

The connector 3 may comprise a first portion 10 coupled to a first needle assembly storage compartment and a second portion 11 coupled to a second needle assembly storage compartment. Exemplary embodiments of the first and second portions 10, 11 are shown in Figure 3. A break point 4 may be formed between the first and second portions 10, 11. Each of the first and second portions 10, 11 may include a narrowed portion 5 which leads to the break point 4.

In an exemplary embodiment, the break point 4 is formed in a second plane substantially perpendicular to the first plane. Thus, the break point 4 facilitates separation of consecutive needle assembly storage compartments 2 when consecutive needle assembly storage compartments 2 are rotated relative in the first plane and/or the second plane about the break point 4. Those of skill in the art will understand that the break point 4 may be formed in any plane and be parallel or disposed at an angle with respect to any plane. Additionally, if there is more than one connector 3 coupled to consecutive needle assembly storage compartments 2, the break points 4 of the connectors 3 may be disposed in the same or different planes.

In the exemplary embodiment shown in Figure 2, each needle assembly storage compartment 2 includes two first portions 10 for coupling to two second portions 11 of a first adjacent needle assembly storage compartment and two second portions 11 for coupling to two first portions 10 of a second needle assembly storage compartment. Thus, in this exemplary embodiment, two connectors 3 are formed between consecutive needle assembly storage compartments. Those of skill in the art will understand that a needle assembly storage compartment may have any number of first and/or second portions, and any number of connectors 3 may be formed between consecutive needle assembly storage compartments.

In the exemplary embodiment shown in Figure 2, the first and second portions 10, 11 of the connectors 3 are formed at a predetermined angle (e.g., 180 degrees) relative to each other on the proximal portion 6 of the needle assembly storage compartments 2, because the array 1 is formed as a one-dimensional row. If a two- or more dimensional array 1 is desired, the first and second portions 10, 11 may be formed at angles relative to each other. For example, if consecutive needle assembly storage compartments 2 are arranged at a 45 degree angle relative to each other, the first and second portions 10, 11 formed on each needle assembly storage compartment 2 may be disposed at a 90 degree relative to each other.

The connectors 3 are shown as connecting the proximal portions 6 of the needle assembly storage compartments 2. Those of skill in the art will understand that a connector 3 may connect any portions of consecutive needle storage compartments 2.

To manufacture the array 1, the needle assembly storage compartments 2 may be formed from a first material (e.g., plastic, rubber, metal, silicon, etc.) and the connectors 3 may be formed from a second material (e.g., polystyrene, polycarbonate, polymethyl methacrylate, etc.). The second material may be characterized as having high rigidity and low ductility.

Figure 3 shows another exemplary embodiment of an array 1 of needle assembly storage compartments 2 according to the present invention. In this exemplary embodiment, consecutive needle assembly storage compartments are inverted relative to each other with the openings 7 of the consecutive needle assembly storage compartments facing in opposite directions. This configuration may enhance a storage density of needle assemblies in a given space, because approximately two times the number of needle assembly storage compartments 2 may be included in this configuration relative to the exemplary configuration depicted in Figure 1.

In this exemplary embodiment, the first portion 10 of the connector 3 may be formed on the proximal portion 6 of the needle assembly storage compartment 2, and the second portion 11 may be formed on distal portion 8 of the needle assembly storage compartment 2. Thus, consecutive needle assembly storage compartments 2 may be connected by a first connector formed between the first portion 10 on the proximal portion 6 of a first needle assembly storage compartment and the second portion 11 on the distal portion 8 of a second needle assembly storage compartment, and a second connector formed between the second portion 11 on the distal portion 8 of the first storage compartment and the first portion 10 on the proximal portion 6 of the second storage compartment.

Those of skill in the art will understand the modifications (additions and/or removals) of various components of the device and/or system and embodiment described herein may be made without departing from the full scope and spirit of the present invention, which encompass such modifications and any and all equivalents thereof.

### List of References

- 1: needle assembly storage array
- 2: needle assembly storage compartment
- 3: connector
- 4: break point
- 5: narrowed portion
- 6: proximal portion
- 7: opening
- 8: distal portion
- 9: seal
- 10: first portion of a connector
- 11: second portion of a connector

## Claims

1. A needle assembly storage array (1) comprising:
a first needle assembly storage compartment;
a second needle assembly storage compartment; and
at least one connector (3) formed between the first needle assembly storage compartment and the second needle assembly storage compartment, wherein the at least one connector (3) includes a break point (4) for separating the first needle assembly storage compartment and the second needle assembly storage compartment.

2. The needle assembly storage array (1) according to claim 1, wherein openings of the first and second needle assembly storage compartments are disposed in a first plane and the break point (4) is disposed in a second plane.

3. The needle assembly storage array (1) according to claim 2, wherein the first plane is substantially perpendicular to the second plane.

4. The needle assembly storage array (1) according to claim 1, wherein the first needle assembly storage compartment is inverted relative to the second needle assembly storage compartment.

5. The needle assembly storage array (1) according to any one of the preceding claims, wherein the at least one connector (3) comprises:
a first portion (10) coupled to the first needle assembly storage compartment; and
a second portion (11) coupled to the second needle assembly storage compartment,
wherein the break point (4) is formed between the first portion (10) and the second portion (11).

6. The needle assembly storage array (1) according to any one of the preceding claims, wherein the at least one connector (3) includes at least one narrowed portion (5) adjacent the break point (4).

7. The needle assembly storage array (1) according to any one of the preceding claims, wherein the first and second needle assembly storage compartments are made of a first material and the at least one connector (3) is made of a second material.

8. The needle assembly storage array (1) according to claim 7, wherein the second material is one of polystyrene, polycarbonate, and polymethyl methacrylate.
